# EUROPEAN PATENT SPECIFICATION

(11) **EP 3 288 546 B1**
(45) Date of publication and mention of the grant of the patent: **15.12.2021**
(21) Application number: 16785701.0
(22) Date of filing: 02.03.2016
(51) Int. Cl.: A61K 31/12, A23K 20/10, A23K 50/75, A61K 31/045, A61P 1/00, A61P 31/04, A61K 31/015, A23K 20/179, A23K 20/105

(54) **OXIDIZED CAROTENOIDS AND COMPONENTS THEREOF FOR PREVENTING NECROTIC ENTERITIS**
OXIDIERTE CAROTINOIDE UND KOMPONENTEN DAVON ZUR VERHINDERUNG VON NEKROTISCHER ENTERITIS
CAROTÉNOÏDES OXYDÉS ET LEURS COMPOSANTS POUR LA PRÉVENTION DE L'ENTÉRITE NÉCROSANTE

(30) Priority: 28.04.2015 US 201562153587 P
(43) Date of publication of application: 07.03.2018
(73) Proprietor: Avivagen Inc., Ottawa ON K1A 0R6 (CA)
(72) Inventor: BURTON, Graham W., Ottawa, Ontario K1W1C2 (CA); GROOME, Cameron L., Mississauga, Ontario L5J 4J9 (CA); NICKERSON, James G., Charlottetown, Prince Edward Island C1A 8S3 (CA); RILEY, William W., Pasig City (PH); DAROSZEWSKI, Janusz, Ottawa, Ontario K1J 6A8 (CA)
(74) Representative: D Young & Co LLP
(86) International application number: PCT/CA2016/050226
(87) International publication number: WO 2016/172787

(56) References cited:
- CA-A1- 2 648 282
- CA-A1- 2 771 204
- CA-A1- 2 840 376
- CA-C- 2 171 625
- JAMES B. JOHNSTON ET AL: "Biologically Active Polymers from Spontaneous Carotenoid Oxidation: A New Frontier in Carotenoid Activity", PLOS ONE, vol. 9, no. 10, 31 October 2014 (2014-10-31), page e111346, XP055498691, DOI: 10.1371/journal.pone.0111346

## Description

### FIELD OF THE INVENTION

This invention relates to the use of fully oxidized beta-carotene ("OxBC"), and compositions and/or products comprising same for preventing and/or reducing the risk of developing Necrotic Enteritis (NE) and ameliorating associated conditions, such as associated gastrointestinal conditions.

### BACKGROUND OF THE INVENTION

### Necrotic Enteritis

Necrotic Enteritis (NE) often occurs in poultry, such as chickens, ducks and turkeys. Most often it occurs in broiler chickens at about 2 - 5 weeks of age or in breeder and layer chickens at about 12 to 16 weeks of age, but may also occur in other animals such as horses, sheep, swine, cattle and fish. It can cause sudden death with or without clinical signs or loss of vitality, reduced feed conversion efficiency, loss of appetite and feed consumption, reduced weight gain or loss of weight, diarrhea, multifocal hyperemia, hemorrhages of small intestine, thin-walled intestine, confluent mucosal necrosis, sudden death, increased mortality, reduced growth rate and uniformity. The major pathogen associated with NE is *Clostridium perfringens.* Predisposing factors include but are not limited to environmental stresses, immunosuppression, concurrent coccidiosis caused by infection with Eimeria species, composition of diet, lack of nutrition, bacteriocins, collagenolytic enzymes & adhesion factors and toxin producing strains, such as *Clostridium perfringens.*

As noted above, necrotic enteritis (NE) has been found in many animals, including various birds, such as poultry birds. It is largely found in commercial broiler chickens when they are produced without the use of antimicrobial growth promoters.

The incidence rate of NE in antibiotic-free broiler production farms has risen from 13.6% in 2008 to about 97.3% in 2011 (H-K Jang, Chonbuk National University).

### Clostridium Perfringens

In healthy broiler chicken populations *C. perfringens* often ranges from 10² to 10⁴ colony forming units (CFU) per gram of the intestinal contents of the small intestine with the levels potentially dependent on one-time or cumulative bacterial challenge. The level of *C*. *perfringens* in NE-affected birds is higher - starting at the rate of healthy birds when beginning to become unbalanced and then moving up logarithmically to sometimes reach 10⁷ colony forming units (CFU) per gram or more by the time they reach market weight.

Current treatments include the use of antibiotics, such as penicillins (e.g. phenoxymethyl penicillin, amoxycillin), which can be placed in drinking water, or Bacitracin in feed (e.g. 100 ppm). Neomycin and erythromycin are also sometimes used . The course of treatment, for instance, in-water medication is commonly 3-5 days and in-feed medication is 5-7 days depending on the severity.

Antibiotics, such as pencillin and bacitracin, have also been used in feed as a preventative.

In many countries local regulations or market conditions prevent the routine use of many antibiotics and the use of antibiotic-free feeds is trending. As such, so is the rise in NE.

There is a need to minimize the effects of NE such as in broiler chickens - for example, by way of protecting them from infection (e.g. to prevent NE) or reducing the levels of *C. perfringens* bacteria.

JAMES B. JOHNSTON ET AL: "Biologically Active Polymers from Spontaneous Carotenoid Oxidation: A New Frontier in Carotenoid Activity", PLOS ONE, vol. 9, no. 10, 31 October 2014 (2014-10-31), page e111346 discloses the effect of oxidized carotenoids, such as OxBC on bacterial pathogens. A specific effect on reducing the severity of intestinal lesions associated with NE is not explicitly disclosed therein.

### SUMMARY OF THE INVENTION

In some aspects, the invention provides a fully oxidized beta-carotene (OxBC) for use in reducing the severity of intestinal lesions associated with Necrotic Enteritis (NE) in poultry, wherein the use comprises administering an effective amount of the fully oxidized beta-carotene to said poultry. In other aspects, the invention provides a composition comprising fully oxidized beta-carotene for use in animal feed to reduce the severity of intestinal lesions associated with Necrotic Enteritis in poultry.

Additional aspects and advantages of the present invention will be apparent in view of the description that follows. It should be understood, however, that the detailed description and the specific examples, while indicating preferred embodiments of the invention, are given by way of illustration only, since various changes and modifications within the scope of the invention will become apparent to those skilled in the art from this detailed description.

### BRIEF DESCRIPTION OF THE FIGURES

The invention will now be described in relation to the drawings, in which:

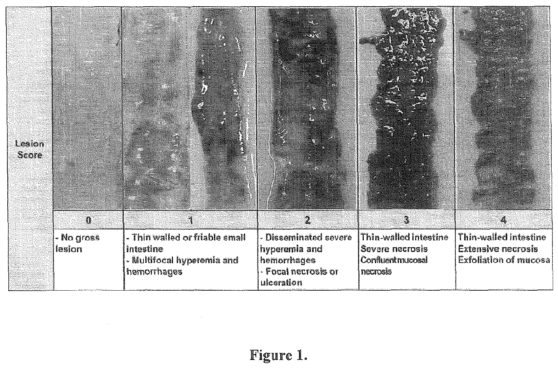

**Figure 1****.** Illustrates macroscopic intestinal lesions resulting from NE, using a known method of scoring their severity, Where: "0" is where there is no gross lesion; "1" is thin walled or friable small intestine and multifocal hyperemia and hemorrhages; "2" is disseminated severe hyperemia and hemorrhages and focal necrosis or ulceration; "3" is thin-walled intestine, severe necrosis confluent mucosal necrosis; and "4" is thin-walled intestine, extensive necrosis, exfoliation of mucosa.

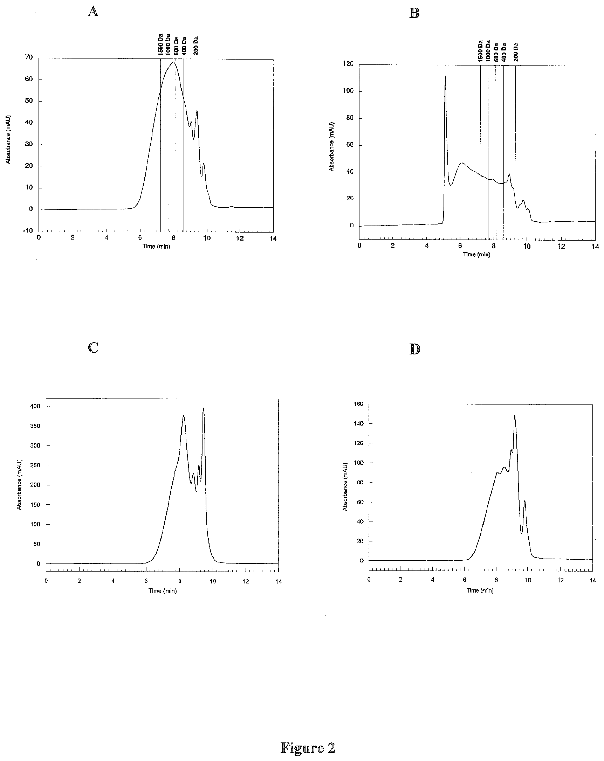

**Figure 2****.** GPC analysis of fully oxidized β-carotene, lycopene, canthaxanthin and lutein. (A) OxBC, (B) OxLyc, (C) OxCan and (D) OxLut. UV absorbance was monitored at 220-400 nm. Where shown, vertical lines correspond to elution times of polyether molecular weight standards (selected markers shown). Column cut-off ca. 10,000 Da. The sharp peak at ca. 5 min in the OxLyc GPC indicates the presence of polymers with molecular weights > 10,000 Da.

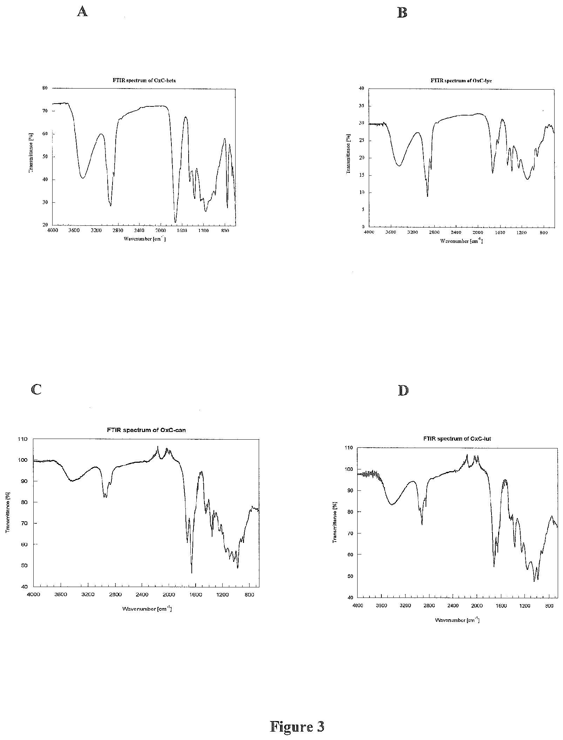

**Figure 3****.** FTIR spectra of (A) OxBC, (B) OxLyc, (C) OxCan and (D) OxLut, obtained using either NaCl disks and a film cast from a CHCl3 solution or KBr disks.

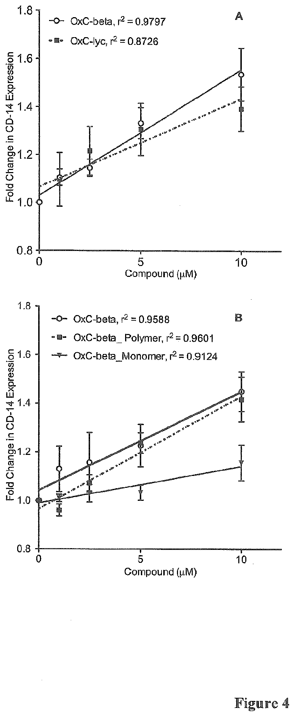

**Figure 4****.** Determination of CD14 receptor expression relative to OxBC of (A) fully oxidized lycopene (OxLyc), and (B) OxBC polymer and norisoprenoid monomer fractions. THP-1 cells were treated for 24 hours with the indicated concentrations of compounds.

### DETAILED DESCRIPTION OF THE INVENTION

**"Animal"** means any animal susceptible to NE, including, without limitation, humans, dogs, cats, horses, sheep, swine, cattle, poultry (including without limitation chickens (such as broiler and layers), ducks and turkeys), and fish.

An **"amount sufficient"** or **"effective amount"** is meant the amount of oxidized carotenoids, such as oxidatively transformed carotenoid or fully oxidized carotenoid or carotenoid-oxygen polymer, or a fractionated component(s) thereof, or compositions comprising same required to prevent and/or enhance the ability to resist NE and associated conditions, recover or overcome same or maintain a healthy state in light of exposure or potential exposure to an NE associated causing agent such as *C. perfringens.* The effective amount of oxidized carotenoid or composition of the invention used to practice the methods of the invention varies depending upon the manner of administration, the type of animal, body weight, and general health of the animal. Ultimately, the attending medical professional such as a physician or veterinarian or animal care professional (including farmers, technicians, those knowledgeable or with skill in the field) will decide the appropriate amount and dosage regimen. Such amount is referred to as an **"amount sufficient"** or **"effective amount".**

**"Associated Conditions of Necrotic Enteritis"** include but are not limited to gastrointestinal symptoms or pathologies, such as reduced appetite, reduced rate of weight gain, reduced feed conversion efficiency or intestinal lesions.

**"Carotenoid"** as used herein refers to naturally-occurring pigments of the terpenoid group that can be found in plants, algae, bacteria, and certain animals, such as birds and shellfish. Carotenoids include but are not limited to carotenes, which are hydrocarbons (i.e., without oxygen), and their oxygenated derivatives (i.e., xanthophylls). Examples of carotenoids include lycopene; α-carotene; γ-carotene; β-carotene; zeaxanthin; echinenone; isozeaxanthin; astaxanthin; canthaxanthin; lutein; citranaxanthin; β-apo-8'-carotenoic acid ethyl ester; hydroxy carotenoids, such as alloxanthin, apocarotenol, astacene, astaxanthin, capsanthin, capsorubin, carotenediols, carotenetriols, carotenols, cryptoxanthin, β-cryptoxanthin, decaprenoxanthin, epilutein, fucoxanthin, hydroxycarotenones, hydroxyechinenones, hydroxylycopene, lutein, lycoxanthin, neurosporine, phytoene, phytofluoene, rhodopin, spheroidene, torulene, violaxanthin, and zeaxanthin; and carboxylic carotenoids, such as apocarotenoic acid, β-apo-8'-carotenoic acid, azafrin, bixin, carboxylcarotenes, crocetin, diapocarotenoic acid, neurosporaxanthin, norbixin, and lycopenoic acid.

**"Carotenoid-Oxygen Copolymer", "Carotenoid Copolymer"** and **"Polymer"** as used herein refers to a carotenoid, which is an unsaturated compound, that has been oxidized at its reactive double bonds by spontaneous reaction with molecular oxygen, resulting in co-polymers of the carotenoid with oxygen as the main product and that is separated and isolated from its norisoprenoid by-products.

**"Fully Oxidized Carotenoid",** as used herein, refers to a carotenoid, which is an unsaturated compound, that has been fully oxidized at its reactive double bonds by spontaneous reaction with molecular oxygen, resulting in a mixture of copolymers of the carotenoid with oxygen and norisoprenoid breakdown products. The fully oxidized carotenoid used in the present invention is OxBC, fully oxidized β-carotene. Other described fully oxidized carotenoids are OxLyc, fully oxidized lycopene; OxLut, fully oxidized lutein; and OxCan, fully oxidized canthaxanthin produced from oxidation of the isolated parent carotenoid referred to as synthetically derived as opposed to naturally sourced products.

**"Fully oxidized beta-carotene"** ("OxBC" - also sometimes referred to in the literature under the Avivagen Inc. OxC-beta^{™} brand) is the complex mixture of products formed by the full, spontaneous oxidation of β-carotene and contains a preponderance of β-carotene-oxygen copolymer compounds. As used herein, it refers to a fully oxidized carotenoid composition derived from the synthetic, commercial product of pure β-carotene, comprising about 85% by weight of β-carotene-oxygen copolymers and about 15% low molecular weight breakdown products called norisoprenoids.

**"Natural"** or **"Naturally Sourced",** as used herein refers to plant sources (including plants or parts thereof, wherein the parts thereof may include but are not limited to seeds, leaves, and stems, fruits or vegetables) or microorganisms. **"Natural Product"** or **"Naturally Sourced Product"** refers to products derived from processing natural sources of oxidized carotenoids, such as carotenoid-oxygen copolymers and compositions comprising same. They are derived from processing natural sources under oxidative polymerization conditions. Such natural sources may include but are not necessarily limited to: carrots, tomato, alfalfa, spirulina, rosehip, sweet pepper, chili pepper, paprika, sweet potato, kale, spinach, seaweed, wheatgrass, marigold, moringa oleifera and red palm oil.

**"Oxidatively Transformed Carotenoid"** refers to a carotenoid which has been reacted with up to 6 to 8 molar equivalents of oxygen, or an equivalent amount of oxygen from another oxidizing agent, resulting in a mixture of very low molecular weight oxidative cleavage products and a large proportion of oligomeric material (i.e., that component of the oxidatively transformed carotenoid having a median molecular weight of about 900 Daltons). The resulting reaction produces a mixture that includes molecular species having molecular weights ranging from about 100 to 8,000 Daltons. The oligomeric material is believed to be formed by the many possible chemical recombinations of the various oxidative fragments that are formed. Methods of making oxidatively transformed carotenoid are described in U.S. Patent No. 5,475,006 and U.S. Patent Application No. 08/527,039.

**"Oxidized Carotenoid"** includes a carotenoid that has been oxidized such as **"Oxidatively Transformed Carotenoid", "Fully Oxidized Carotenoid"** and **"Carotenoid-Oxygen Copolymer"** and components thereof that result in desired activities, compositions and products containing same, such as Carotenoid-Oxygen Copolymer containing products and compositions.

**"Pharmaceutical Composition"** is meant a composition containing oxidized carotenoids, oxidatively transformed carotenoid, fully oxidized carotenoid or a fractionated component thereof or a carotenoid-oxygen copolymer or carotenoid-oxygen copolymer containing product, and formulated with one or more pharmaceutical-grade excipients in a manner that conforms with the requirements of a governmental agency regulating the manufacture and sale of pharmaceuticals as part of a therapeutic regimen for the treatment or prevention of disease in a mammal (e.g., manufactured according to GMP regulations and suitable for administration to a human). Pharmaceutical compositions can be formulated, for example, for oral administration in unit dosage form (e.g., a tablet, capsule, caplet, gelcap, or syrup); for topical administration (e.g., as a cream, gel, lotion, or ointment); for intravenous administration (e.g., as a sterile solution free of particulate emboli and in a solvent system suitable for intravenous use); or any other formulation described herein.

**"Provitamin A Carotenoids"** or **"PVA"** refer to those carotenoids, namely α-, β- and γ-carotenes and β-cryptoxanthin, that are capable of being converted by oxidation into vitamin A. **"OxPVA"** refers to oxidized provitamin A carotenoids.

**"Treating"** refers to administering a composition for prophylactic and/or therapeutic purposes. To **"prevent disease"** refers to prophylactic treatment of an animal who is not yet ill, but who is susceptible to, or otherwise at risk of, a particular disease. To **"treat disease"** or use for **"therapeutic treatment"** refers to administering treatment to an animal already suffering from a disease to improve or stabilize the animal's condition. Thus, in the claims and embodiments, treating is the administration to an animal either for therapeutic or prophylactic purposes. Therapy according to the invention may be performed alone or in conjunction with another therapy. As used herein, **"at risk"** refers to animals prone to poor health, disease, susceptibility to infections, joint mobility issues, reduced activity levels, and/or poor coat quality.

### Oxidized Carotenoids

Various health benefits are ascribed to dietary carotenoids. The several provitamin A carotenoids, including α- and β-carotenes and β-cryptoxanthin, provide benefits linked to their vitamin A activities. However, less easily explained are other, non-vitamin A benefits of both provitamin A carotenoids and of other carotenoids that cannot be converted into vitamin A.

Carotenoids are yellow, orange, and red pigments synthesized by plants. There are over 600 known carotenoids that are made up of two classes called carotenes, which are purely hydrocarbons, and xanthophylls, which are carotenes substituted with one or a few oxygen atoms. β-carotene, and lycopene are examples of common carotenes, whereas lutein, zeaxanthin, and canthaxanthin are common examples of xanthophylls. The most common carotenoids in North American diets are α-carotene, β-carotene, β-cryptoxanthin, lutein, zeaxanthin, and lycopene.

All carotenoids are formed from 8 isoprene units and each carotenoid molecule contains 40 carbon atoms. Structurally, carotenoids take the form of a polyene hydrocarbon chain, which is sometimes terminated at one or both ends by a ring. Carotenoids that contain unsubstituted β-ionone rings (including β-carotene, α-carotene, β-cryptoxanthin and γ-carotene) have vitamin A activity (meaning that they can be converted to retinal). By contrast, lutein, zeaxanthin, and lycopene have no vitamin A activity.

Traditionally, non-vitamin A activities have been ascribed to actions of the carotenoid itself, often as an antioxidant. However, recent research casts doubt upon an antioxidant role, at least with regard to inhibiting carcinogenesis, and points to the operation of other mechanisms.

Although it has been long known that addition of oxygen is inherently favored in spontaneous oxidation of highly unsaturated compounds, the predominant involvement and the significance of oxidative polymerization of carotenoids had surprisingly escaped notice prior to the inventors' reports (also see US 5,475,006; US 7,132,458; US 8,211,461; US 2011-0217244; US 2013-0131183; and US 2013-0156816). Furthermore, the studies with a fully-oxidized β-carotene composition (termed OxBC, the active ingredient in Avivagen Inc.'s OxC-beta^{™} branded products) obtained by spontaneous reaction of β-carotene with oxygen in a solvent as well with the analogously formed fully oxidized lycopene, have revealed that the polymeric fraction is responsible for immunological activity, which includes an ability to prime and enhance innate immune function as well as to limit inflammatory processes. The extended system of linear conjugated double bonds present in β-carotene is common to all carotenoids so it is expected that other carotenoids will behave similarly in their spontaneous reactions with molecular oxygen and may explain the non-vitamin A effects of both the provitamin A carotenoids (α-, β- and γ-carotenes and β-cryptoxanthin) and the more numerous carotenoids that cannot be converted into vitamin A.

Further, given the ubiquity of carotenoids, including and especially β-carotene, and their known susceptibility to loss during processing of food, it is unclear whether and to what extent oxidation and, in particular, copolymerization occur naturally in foods and may account for this loss.

The inventors' discovery of food (such as plant sources) containing carotenoid-oxygen copolymers, as disclosed herein, with anticipated non-vitamin A immunological activities. For instance, in one example as described herein, the chemical nature of the compound isolated from carrot powder (originally rich in β-carotene) was confirmed by comparing elemental analysis, GPC, IR, GC-MS thermolysis and UV data with those from OxBC. Elemental analysis, IR and GPC data of compounds isolated in the same manner from other dried foods supported their oxygen-copolymer nature.

Geronic acid, a low molecular weight marker of the presence of β-carotene-oxygen copolymers, occurs in common fresh or dried foods, including carrots, tomatoes, sweet potatoes, paprika, rosehips, seaweeds, alfalfa and milk, at levels encompassing an approximately thousand-fold range, from low parts-per-billion in fresh foods to parts-per-million in dried foods. Copolymers isolated from some dried foods, e.g. carrot powder, tomato powder, spirulina powder, rosehip powder, paprika powder, seaweed powder, and wheatgrass powder, reach parts-per-thousand levels - comparable to the original carotenoid levels. *In vivo* biological activity of supplemental β-carotene-oxygen copolymers has been previously documented at parts-per-million levels, suggesting certain foods have such activity.

The finding of the present inventors that oxidation and the associated reaction products would be found within the much more complex environment in which carotenoids occur naturally, namely in certain plant sources, such as fruits and vegetables and certain microorganisms (algae, fungi and bacteria) was not obvious or predictable in light of the complex micro-environment and the many other potentially reactive compounds in the biological material that could divert any incipient carotenoid oxidation reaction down a myriad of other pathways with different product outcomes.

In one described example, the carotenoid-oxygen copolymer products isolated in this manner from dried plant-derived foods do *not* contain the other anticipated low molecular weight carotenoid breakdown products (e.g., including geronic acid in products expected to contain β-carotene oxidation breakdown compounds). This is distinct from fully oxidized carotenoids (such as, OxBC, OxLyc, OxLut or OxCan), which do contain such products.

In another example, the plant source is selected from the group consisting of: carrots, tomato, alfalfa, spirulina, rosehip, sweet pepper, chili pepper, paprika, sweet potato, kale, spinach, seaweed, wheatgrass, marigold, moringa oleifera and red palm oil. In another embodiment, the sources are plant products that are powders, e.g. carrot powder, tomato powder, spirulina powder, rosehip powder, paprika powder, seaweed powder, and wheatgrass powder.

In one example, the microorganism source is selected from the group consisting of: bacteria, yeast, fungi, and algae, such as spirulina⁴⁴ and genetically modified forms of same that enhance carotenoids and therefore the potential for carotenoid-oxygen copolymers. In some further embodiments, the microorganisms are selected from the group of the following species: Algae: Spirulina, Dunaliella, Haematococcus, Murielopsis. Fungi: Blakeslea trispora. Yeasts: Xanthophyllomyces dendrorhous, Rhodotorula glutinis. Bacteria: Sphingomonas.

The inventors' discovery that OxBC β-carotene-oxygen copolymer compounds as used in the Examples are beneficial in the uses, methods, compositions and kits described herein leads to the expectation that copolymer counterparts in foods will impart bioactivities with similar health implications. *In situ* oxidation of dietary carotenoids resulting from oxidative processes unleashed during digestion of fruit or vegetables also could at least partially account for the variable and several-fold lower vitamin A activity of β-carotene in foods compared to β-carotene from supplements. Oxidative destruction of β-carotene and a perceived loss of activity could actually be a gain of immunological activity through copolymer formation.

The present inventors have developed a way to enhance the amount of carotenoid-oxygen copolymer in a source and/or to have a source with known and consistent amounts of carotenoid-oxygen copolymer to facilitate consistent dosing to known effective amounts to achieve desired results.

Further, the present invention enables one to produce carotenoid oxygen copolymer comprising products *in situ* without starting from isolated carotenoids as the source and to provide products comprising consistent levels of carotenoid-oxygen copolymers which have resulting animal benefits described herein including relating to NE. In one aspect the carotenoid-oxygen copolymers from natural sources do not comprise (or minimally comprise) breakdown products.

### Methods and Uses

As described herein, the examples have several applications regarding the use of oxidized carotenoids or oxidized carotenoid containing compositions or products, such as oxidatively transformed carotenoids, fully oxidized carotenoids (such as OxBC), components thereof or fractionated components thereof, such as components comprising a carotenoid-oxygen copolymer containing component. They include but are not limited to the following applications:

### A. Weight Gain Restoration.

The described example illustrates that oxidized carotenoids or oxidized carotenoid containing compositions or products, such as oxidatively transformed carotenoids, fully oxidized carotenoids (such as OxBC), components thereof or fractionated components thereof, such as components comprising a carotenoid-oxygen copolymer containing component, such as OxBC can be used to improve final mean body weight at low parts per million (ppm) levels in feed, with restoration to the level of the non-challenged bird group. In one described example, the invention provides a method for using oxidized carotenoids or oxidized carotenoid containing compositions or products, such as oxidatively transformed carotenoids, fully oxidized carotenoids (such as OxBC), components thereof or fractionated components thereof, such as components comprising a carotenoid-oxygen copolymer containing component, such as OxBC to protect against productivity losses caused by sub-clinical disease burdens associated with *C. perfringens* and/or NE, in broiler chickens. One can use oxidized carotenoids or oxidized carotenoid containing compositions or products, such as oxidatively transformed carotenoids, fully oxidized carotenoids (such as OxBC), components thereof or fractionated components thereof, such as components comprising a carotenoid-oxygen copolymer containing component, such as OxBC as described herein to "restore" weight gain to the level that would have resulted had there been no disease, NE or *C. perfringens* infection.

### B. Intestinal Lesion Protection.

Intestinal lesion scores due to the NE infection were significantly alleviated in the OxBC treatment groups compared to the non-medicated bird group, with the 2 ppm OxBC group showing the most improvement. Other oxidized carotenoids or oxidized carotenoid containing compositions or products, such as oxidatively transformed carotenoids, fully oxidized carotenoids (such as OxBC), components thereof or fractionated components thereof, such as components comprising a carotenoid-oxygen copolymer containing component could similarly be used.

As such, in one aspect, the invention provides fully oxidized beta-carotene for use in reducing the severity of intestinal lesions associated with Necrotic Enteritis (NE) in poultry, wherein the use comprises administering an effective amount of the fully oxidized beta-carotene to said poultry. The invention also provides a composition comprising fully oxidized beta-carotene for use in animal feed to reduce the severity of intestinal lesions associated with Necrotic Enteritis in poultry.

### C. Pathogen Load Reduction.

The number of *C. perfringens* bacteria in feces was reduced by OxBC, in a dose-dependent pattern. Thus the invention can be used to maintain the balance of bacteria in the gut, such as the gut of a broiler chicken, and prevent overgrowth of the bacteria *C. perfringens.*

### D. Prevention & Improvement of NE.

Low ppm levels of oxidized carotenoids or oxidized carotenoid containing compositions or products, such as oxidatively transformed carotenoids, fully oxidized carotenoids (such as OxBC), components thereof or fractionated components thereof, such as components comprising a carotenoid-oxygen copolymer containing component, or in one embodiment OxBC, in feed can contribute to the prevention and improvement of NE in commercial broiler chicken farming and is expected to have a positive effect in improving productivity for the feeding period.

### E. Use of oxidized carotenoids or oxidized carotenoid containing compositions or products, such as oxidatively transformed carotenoids, fully oxidized carotenoids (such as OxBC), components thereof or fractionated components thereof, such as components comprising a carotenoid-oxygen copolymer containing component instead of antibiotics to control NE and C. perfringens infection.

In one example provided is a method to maintain poultry (such as boiler chickens) in good health (such as a non-challenged health status) in the face of a challenge, such as NE and *C. perfringens,* using oxidized carotenoids or oxidized carotenoid containing compositions or products, such as oxidatively transformed carotenoids, fully oxidized carotenoids (such as OxBC), components thereof or fractionated components thereof, such as components comprising a carotenoid-oxygen copolymer containing component instead of an antimicrobial. The use of antimicrobials in poultry livestock has been widely associated with drug residues in food and the propagation of antibiotic-resistant pathogens in food and the environment. Accordingly there is pressure on poultry producers to reduce their usage of antibiotics. Oxidized carotenoids or oxidized carotenoid containing compositions or products, such as oxidatively transformed carotenoids, fully oxidized carotenoids (such as OxBC), components thereof or fractionated components thereof, such as components comprising a carotenoid-oxygen copolymer containing component. In one embodiment OxBC has demonstrated herein that it has neither bactericidal nor bacteriostatic properties and is therefore not an antimicrobial, making it a useful alternative to antibiotics for the prevention or control of NE and *C. perfringens* in poultry, such as boiler chickens.

### Animals

The described method can be used in animals, particularly poultry and in another embodiment, broiler chickens, layers and turkeys, that are susceptible to *C. perfringens* challenges and/or NE

### Oxidized Carotenoids, such as OxBC, Compositions and Modes of Administration and Kits

OxBC is the product of the full autoxidation of beta-carotene, a process that results in a novel composition free of beta-carotene, vitamin A or retinoic acid receptor agonist activity. It is predominantly formed by an oxygen copolymerization process that leads to a well-defined, consistently-reproducible product comprised largely of β-carotene-oxygen copolymers.

Structurally, OxBC polymers appear to be a less polymerized form of sporopollenin, a biopolymer found in the exine walls of spores and pollens. The polymers have since been identified as being formed from the autoxidation of multiple carotenoids, including not only beta-carotene, but also, canthaxanthin, lutein and lycopene. Other carotenoids such as astaxanthin are fully expected to predominantly undergo the same polymerization process.

Chemical analyses indicate that the synthesized version is highly analogous to polymers naturally occurring in different foods, feeds and forages (unpublished data; manuscript in preparation).

OxBC is produced in quantity by heating a solution of synthetic beta-carotene in a "GRAS" solvent, in an atmosphere of pure oxygen until oxygen uptake becomes very much slower as a defined endpoint is reached. Manufacturing QC/QA methods have been established and production capacity of the pure active (currently targeted to in-feed livestock applications) is now in the tens of metric tons per year. OxBC was supplied for use in broilers as a 10% concentrate produced by spray-drying a solution of OxBC onto corn starch to form a consistent "pre-mix" product. Greater or lesser concentrations of the active ingredient might be used to mill into feeds or other dosing preparations at the desired levels of administration. Other Fully Oxidized Carotenoids can be prepared similarly.

OxBC can, as well as other oxidized carotenoids, be formulated together with feed or be administered separately from the feed, contemporaneously or not.

In one embodiment, the OxBC or OxBC containing composition is administered or fed in an effective amount over the animal's free intake of food. In one embodiment, the OxBC or OxBC containing composition is administered or fed freely or at intervals or in another embodiment, once a day, in another embodiment twice per day, in yet another embodiment several times a day and wherein the amount of OxBC is adjusted to the desired amount depending on feeding patterns of the animals.

In one embodiment the amount of OxBC is 2 ppm to 30 ppm or in another embodiment, 2 to 15 ppm, to 10 ppm, to 8 ppm, or to 6 ppm, provided over short intervals. Another, preferred embodiment suggests from experimental results that 2 ppm to 4 ppm fed daily over a longer interval may provide the optimal effect while preserving cost-effectiveness for the poultry producer. PPM refers to parts of active ingredient (e.g., oxidized carotenoid) per million parts of diet or feed.

OxBC can be administered by any means that produce contact of said active agent with the agent's sites of action in the body of a subject or patient to produce a therapeutic effect, in particular a beneficial effect, more particularly a sustained beneficial effect. The active ingredients can be administered simultaneously or sequentially and in any order at different points in time to provide the desired beneficial effects. A compound and composition of the invention can be formulated for sustained release, for delivery locally or systemically. It lies within the capability of a skilled physician or veterinarian to select a form and route of administration that optimizes the effects of the compositions and treatments of the present invention to provide therapeutic effects, in particular beneficial effects, more particularly sustained beneficial effects.

In one embodiment, administration of OxBC includes any mode that produces contact of said active agent with the agent's sites of action *in vivo* or in the body of the broiler chicken or other animal, to produce the desired or therapeutic effect, as the case may be. As such it includes administration of OxBC directly or through a mode of delivery e.g. sustained release formulations, delivery vehicles that result in site-directed delivery to the gut or desired site in the body. In one embodiment, the OxBC or OxBC containing compositions can be used in about 1-30 ppm as one embodiment, in another embodiment 2 weeks at 30 ppm, in another embodiment of 2-6 ppm for 'continuous use' (inclusion in a normal diet), or as otherwise described herein. The above-described substances may be formulated into suitable compositions for administration to the animals, such as broiler chickens, in a biologically compatible form suitable for administration *in vivo.* By "biologically compatible form suitable for administration *in vivo"* is meant a form of the substance to be administered in which any toxic effects are outweighed by the therapeutic effects. The substances may be administered to living organisms including animals such as poultry, in another embodiment chickens, in another embodiment broiler chickens, layers or turkeys.

Thus one example provides the use of oxidized carotenoids or oxidized carotenoid containing compositions or products, such as oxidatively transformed carotenoids, fully oxidized carotenoids (such as OxBC), components thereof or fractionated components thereof, such as components comprising a carotenoid-oxygen copolymer containing component in the preparation of a medicament for the applications noted herein. In one embodiment, a therapeutically effective amount of OxBC or a pharmaceutical composition as described herein is administered to a patient in need thereof. A patient in need thereof is a poultry animal that may benefit from OxBC or OxBC containing compositions and its effects as described herein.

An active substance may be administered in a convenient manner such as by injection (subcutaneous, intravenous, etc.), oral administration, inhalation, mucosal application, topical application, transdermal application, gastric application, enteric application or rectal administration. Depending on the route of administration, the active substance may be coated in a material to protect the compound from the action of enzymes, acids and other natural conditions that may inactivate the compound. In one embodiment, OxBC or OxBC containing compositions are administered through the feed of an animal or using other techniques known in the art.

The compositions described herein can be prepared by *per se* known methods for the preparation of pharmaceutical acceptable compositions which can be administered to subjects, such that an effective quantity of the active substance is combined in a mixture with a pharmaceutical acceptable vehicle or carrier. Suitable vehicles or carriers are described, for example, in Remington's Pharmaceutical Sciences (Remington's Pharmaceutical Sciences, Mack Publishing Company, Easton, Pa. , USA 1985 or Remington's The Sciences and Practice of Pharmacy, 21st Edition", (University of the Sciences in Philadelphia, 2005) or Handbook of Pharmaceutical Additives (compiled by Michael and Irene Ash, Gower Publishing Limited, Aldershot, England (1995)). On this basis, the compositions include, albeit not exclusively, solutions of the substances in association with one or more pharmaceutical acceptable vehicles, carriers or diluents, and may be contained in buffered solutions with a suitable pH and/or be iso-osmotic with physiological fluids. In this regard, reference can be made to U. S. Patent No. 5,843,456.

### Feeds and Foodstuffs, Supplements and Pre-Mixes

In one embodiment the invention provides feeds (poultry feeds) or foodstuffs comprising OxBC or OxBC containing compositions, which can be admixed with a foodstuff and fed to the animal in an amount effective for reduce the severity of intestinal lesions associated with Necrotic Enteritis in poultry.

In preparing a foodstuff of the invention, the OxBC or OxBC containing composition is optionally admixed with a bulking agent prior to being added to the foodstuff. Bulking agents include, without limitation, starch, protein, fats, and mixtures thereof. Desirably, the bulking agent is selected from corn starch, whey, flour, sugar, soybean meal, maltodextrin, and guar gum. Foodstuffs of the invention can also include antioxidants to prevent further oxidation of the oxidatively transformed carotenoid or a component thereof. Oxidation can be prevented by the introduction of naturally-occurring antioxidants, such as vitamin E, vitamin C, and tocopherol or of synthetic antioxidants such as butylated hydroxytoluene, butylated hydroxyanisole, tertiary-butylhydroquinone, propyl gallate or ethoxyquin to the foodstuff. The amount of antioxidants incorporated in this manner depends on requirements such as product formulation, shipping conditions, packaging methods, and desired shelf-life.

Foodstuffs of the invention is a poultry feed (e.g., turkey poultry feed, broilers poultry feed, or breeders poultry feed.

The animal feeds are generally formulated to provide nutrients in accordance with industry standards. The feeds may be formulated from a variety of different feed ingredients, which are chosen according to market price and availability. Accordingly, some components of the feed may change over time. For discussions on animal feed formulations and NRC guidelines, see Church, Livestock Feeds and Feeding, O&B Books, Inc., Corvallis Oreg. (1984) and Feeds and Nutrition Digest, Ensminger, Oldfield and Heineman eds., Ensminger Publishing Corporation, Clovis, Calif. (1990).

Other ingredients may be added to the animal feed as needed to promote the health and growth of the animal. The ingredients include, without limitation, sugars, complex carbohydrates, amino acids (e.g., arginine, histidine, isoleucine, leucine, lysine, methionine, phenylalanine, threonine, tryptophan, valine, tyrosine, alanine, aspartic acid, sodium glutamate, glycine, proline, serine, and cysteine, among others), vitamins (e.g., thiamine, riboflavin, pyridoxine, niacin, niacinamide, inositol, choline chloride, calcium pantothenate, biotin, folic acid, ascorbic acid, and vitamins A, B, K, D, E, among others), minerals, protein (e.g., meat meal, fish meal, liquid or powdered egg, fish solubles, whey protein concentrate), oils (e.g., soybean oil), cornstarch, calcium, inorganic phosphate, copper sulfate, and sodium chloride. Any medicament ingredients known in the art may also be added to the animal feed, including, without limitation, antibiotics and hormones. For vitamin, mineral and antibiotic supplementation of animal feeds see Church, Livestock Feeds and Feeding, O&B Books, Inc., Corvallis Oreg. (1984).

Any animal feed blend known in the art can be used in accordance with the present invention, including, without limitation, forages, such as orchard grass, timothy, tall fescue, ryegrass, alfalfa, sainfoin, clovers and vetches, grain feeds, such as corn, wheat, barley sorghum, triticale, rye, canola, and soya beans, crop residues, cereal grains, legume by-products, and other agricultural by-products. In situations where the resulting feed is to be processed or preserved, the feed may be treated with oxidatively transformed carotenoid, a component thereof, or fractionated oxidatively transformed carotenoid before processing or preservation. Desirably, the animal feed of the invention includes rapeseed meal, cottonseed meal, soybean meal, or cornmeal.

Processing may include drying, ensiling, chopping, pelleting, cubing, baling, rolling, tempering, grinding, cracking, popping, extruding, micronizing, roasting, flaking, cooking, and/or exploding. For example, pelleted feed is created by first mixing feed components and then compacting and extruding the feed components through a die with heat and pressure. Animal feeds of the invention can be pelleted as described in, for example, MacBain, Pelleting Animal Feed, American Feed Manufacturers Association, Arlington, Va. (1974).

The present invention is described in the following Examples, which are set forth to aid in the understanding of the invention, and should not be construed to limit in any way the scope of the invention as defined in the claims which follow thereafter.

### EXAMPLES

### EXAMPLE 1 - Preparation of Fully Oxidized Beta Carotene (OxBC)

OxBC is the product of the full autoxidation of beta-carotene, a process that results in a composition free of beta-carotene, vitamin A or retinoic acid receptor agonist activity. It is predominantly formed by an oxygen copolymerization process that leads to a well-defined, consistently reproducible product comprised largely of β-carotene-oxygen copolymers that is more fully described in Burton et al. (1995 and 2014). Structurally, OxBC polymers appear to be a less polymerized form of sporopollenin, a biopolymer found in the exine walls of spores and pollens.

### EXAMPLE 2 - Preparation of Animal Feeds with OxBC

The Animal Feeds used in this invention, prepared by incorporating OxBC as a 10% (w/w) premix with the other feed ingredients, contained the following:

**Table 1. Formula of basal commercial feed used in this study**

| Quantity (v/w) of feed component | | Category | |
|---|---|---|---|
| | | Feed-A (crumb form) | Feed-B (pellet form) |
| | Crude protein | 21.00% | 21.00% |
| | Crude fat | 5.00% | 5.00% |
| | Crude fiber | 6.00% | 6.00% |
| | Crude ash | 8.00% | 8.00% |
| | Calcium | 0.90% | 0.85% |
| | Phosphorus | 1.00% | 1.00% |
| | M+C | 0.90% | 0.85% |
| | MEn | 3.08% | 3.10% |
| | Use period | Before 3 weeks | After 3 weeks |

Substances used:
- OxBC (Avivagen, Canada), groups 3, 4, and 5 (Table 4)Bacitracin (BS Bacitrex 100, Korea), group 6 (Table 4)
- Virginiamycin (Stafac-20, Bayer Korea, Korea), group 7 (Table 4)
- Substances were added to the basal commercial diet as indicated in Table 4

### EXAMPLE 3 - Preventive Effect of OxBC on Necrotic Enteritis Challenge Model with Broiler Chicken

Necrotic enteritis (NE) has been largely found in commercial broiler chickens when they are produced without the use of antimicrobial growth promoters. This referenced study was designed to evaluate the preventive effect of OxBC ('OxC-beta^{™} Livestock product from Avivagen Inc., Canada) in a model of subclinical necrotic enteritis in broiler chickens. OxBC was evaluated for its ability to favorably effect various measures of broiler health and productivity, including survival (mortality) rate, clinical signs, body weight, weight gain, intestinal lesions, and bacterial enumeration compared to a challenged-non-medicated control group.

### Materials and Methods:

### 1. Animals

A total of 280, one-day old Ross broiler chicks, obtained from a commercial hatchery, were used in this study. Chicks were vaccinated for Newcastle disease (Newcastle disease virus) at the hatchery.

### 2. Bird housing and Installation

Birds were reared in isolator units during the entire experimental period.
- Chicken isolators equipped with ventilation system were used in this study.
- There was 1 isolator unit per treatment group and each isolator unit housed twenty chicks on study day-1 (Table 1). The study was run as two parallel replicates with 7 isolator units per replicate.
- The indoor temperature of the isolator was adjusted in order to maintain the optimal temperatures for broilers.
- On study day-1 there were 20 chicks per isolator. On study day-10 the five smallest chicks in each isolator were euthanized and evaluated for gross lesions of the small intestine and enumeration *C. perfringens* in gut content. The evaluation at day-10 was done to ensure the absence of NE lesion or pathogenic levels of *C. perfringens* prior to experimental challenge on day-14. After study day-10 there were 15 birds in each isolator and these birds were used in the challenge portion of the study.
- Chickens were evaluated and weighed individually, therefore each bird represents an experimental unit.

**Table 2. Distribution of starting body weights**

| **Range of body weight (g)** | **<40^{a}** | **41∼43** | **44∼46** | **47∼49** | **50∼52** | **Total^{b}** |
|---|---|---|---|---|---|---|
| No. (%) of chicks | 98 | 75 | 105 | 105 | 17 | 400 |
| | (24.5) | (18.8) | (26.3) | (26.3) | (4.3) | (100) |
| No. of chicks used in this trial per group | 0 | 5 | 7 | 7 | 1 | 20 |
| Total No. | 0 | 70 | 98 | 98 | 14 | 280 |

| | | | | | | |
|---|---|---|---|---|---|---|
| ^{a} Chicks weighing less than 40 g were excluded due to the high mortality rate anticipated for underweight chicks. ^{b} 280 of a total of 400 chicks were used in the experiment and the number of chicks per group on day-1 was 20 . | | | | | | |

### 3. Feeding

The basal diet used in the study was a standard commercial broiler feed. The basal diet was supplemented with the appropriate levels of OxBC or antibiotic as indicated in Table 4. No in-feed anti-coccidials were used in the study. The subclinical necrotic enteritis chicken model, was induced by challenge with *C. perfringens* (CP-13) isolated from a field case of necrotic enteritis in broiler chicken. Challenge was done by oral gavage two times a day with approximately 1 × 10⁷ CFU/ml on days 14, 15, and 16. In one embodiment, a 10% premix of OxBC on starch was incorporated in the feed along with other ingredients.

### • Feeding program

▪ The inventors applied the same feeding program and formulation as in the commercial broiler chicken farms (Table 1).
▪ The experimental feeds were given continuously during the 28-day study period and water was given *ad libitum* throughout the whole experiment.

The formula of basal commercial feed used in this study can be seen in Table 1 above.

### • Challenge

▪ Application of *Clostridium perfringens* CP-013 strain to induce a subclinical NE model.
▪ All birds except Group 1 were orally challenged two times a day with approximately 10⁷ CFU/ml *C. perfringens* on days 14, 15 and 16 (Table 3).
▪ The challenge strain possessed cpa and cpb2 genes, but was negative for the netB toxin gene.
▪ See Shojadoost et al., 2012 for detailed description of methods for inducing NE in poultry.

**Table 3. Clostridium perfringens challenge administration**

| Date | Time | CFU/ml |
|---|---|---|
| Study day-14 | 10:00 | 3.9×10⁷ |
| | 15:00 | 2.1×10⁷ |
| Study day-15 | 10:00 | 3.4×10⁷ |
| | 15:00 | 1.8×10⁷ |
| Study day-16 | 10:00 | 3.6×10⁷ |
| | 15:00 | 1.6×10⁷ |

### • Macroscopic lesion scoring

▪ Gross (macroscopic) lesion scores were assessed using the criteria of Prescott (1978), with scores ranging from a scale of 0 (no gross lesion) to 4 (most severe gross)
▪ Gross lesions in the intestinal tract were graded as follows: 0, no gross lesions; 1+, thin-walled or friable small intestine; 2+, focal necrosis; 3+, larger patches of necrosis; 4+, severe, extensive necrosis typical of field cases (Figure 1).
▪ See Prescott *et al.,* 1978 for a detailed description of the lesion scoring scale.

### • Bacterial enumeration

▪ The number of *Clostridium perfringens* was measured as colony forming units per one gram feces collected from the small intestine after necropsy.
▪ Briefly, 250 µL of fecal sample was loaded into the first well of each row in a 96-well plate, and 10-fold serial dilutions were made using a multichannel pipette by transferring 20 µL from column into 180 µL of medium in column, mixing 10 times, and repeating the process; pipette tips were changed between dilutions.
▪ Thereafter, five replicates of 10 µL from each of the five selected dilutions were plated onto an agar medium using a multichannel pipette.
▪ Plates were allowed to dry, and then placed into an incubator.
▪ See Chen *et al.,* 2003 for a detailed description of the bacterial enumeration methods.

### • Experimental design & Parameters (See Tables 4 and 5)

**Table 4. Experimental design (in parallel relicates)^{a}**

| **Groups** | **No. of birds**^{b} | **Substances** | **Dose** | **Dosage period** | **Challenge strain** | **Period** |
|---|---|---|---|---|---|---|
| G1 | 15 | Non-challenged control | - | No additives | | 28 days |
| G2 | 15 | Non-medicated control | - | No additives | *C. perfringens:* CP-013 (Oral gavage) | |
| G3 | 15 | OxBC | 2 ppm | Days 1 - 28, entire study period. | | |
| G4 | 15 | OxBC | 4 ppm | | | |
| G5 | 15 | OxBC | 6 ppm | | | |
| G6 | 15 | Bacitracin | 55 ppm | | | |
| G7 | 15 | Virginiamycin | 2 ppm | | | |

| | | | | | | |
|---|---|---|---|---|---|---|
| ^{a} The study was conducted in two parallel replicates with each replicate consisting of 7 isolator units. ^{b} On study day-1 there were 20 chicks per isolator, 5 chicks were removed on study day-10 for pre-challenge evaluation of NE markers. | | | | | | |

**Table 5. Evaluation parameters**

| **No.** | **Parameters** | **Definition** | **Period** |
|---|---|---|---|
| 1 | Survival rate | No. of survival bird / no. of total bird | At 28 days old |
| 2 | Clinical signs | Severe depression, decreased appetite, reluctance to move, diarrhea, and ruffled feathers | During 14-to-28 day periods |
| 3 | Mean body weight &Weight gain (%) | % = [(Final Bodyweight (BW) - Initial BW) /Initial BW] × 100 | At 14, 21 & 28 days old |
| 4 | Macroscopic lesion score | Mean value of individual birds | At 21 & 28 day s old |
| 5 | Microscopic lesion score | Mean value of individual birds | At 21 & 28 days old |
| 6 | CFU/g score | Colony forming unit (CFU) /gram of feces | At 21 & 28 days old |
| 7 | Protection rate | Final results to some parameters | At 28 days old |

### Results:

### 3.1 Survival rate (Mortality) & Clinical signs

- Following the CP challenge (days 14-16), no mortality (acutely dead) or clinical signs (severe depression, decreased appetite, reluctance to move, diarrhea, and ruffled feathers) were observed in any birds (See Tables 6 and 7).

**Table 6. Survival rate**

| **Groups** | | **No. (%) of survival birds** | | |
|---|---|---|---|---|
| | | **Replicate 1 (n=15)** | **Replicate 2 (n=15)** | **Mean (n=30)** |
| G1 | Non-challenged control | 15/15 (100) | 15/15 (100) | 30/30 (100) |
| G2 | Non-medicated control | 15/15 (100) | 15/15 (100) | 30/30 (100) |
| G3 | OxBC 2ppm | 15/15 (100) | 15/15 (100) | 30/30 (100) |
| G4 | OxBC 4ppm | 15/15 (100) | 15/15 (100) | 30/30 (100) |
| G5 | OxBC 6ppm | 15/15 (100) | 15/15 (100) | 30/30 (100) |
| G6 | Bacitracin 55ppm | 15/15 (100) | 15/15 (100) | 30/30 (100) |
| G7 | Virginiamycin 2ppm | 15/15 (100) | 15/15 (100) | 30/30 (100) |

**Table 7. Clinical signs**

| **Groups** | | **No. (%) of birds with clinical sign** | | |
|---|---|---|---|---|
| | | **Replicate 1 (n=15)** | **Replicate 2 (n=15)** | **Mean (n=30)** |
| G1 | Non-challenged control | 0/15 (0) | 0/15 (0) | 0/30 (0) |
| G2 | Non-medicated control | 0/15 (0) | 0/15 (0) | 0/30 (0) |
| G3 | OxBC 2ppm | 0/15 (0) | 0/15 (0) | 0/30 (0) |
| G4 | OxBC 4ppm | 0/15 (0) | 0/15 (0) | 0/30 (0) |
| G5 | OxBC 6ppm | 0/15 (0) | 0/15 (0) | 0/30 (0) |
| G6 | Bacitracin 55ppm | 0/15 (0) | 0/15 (0) | 0/30 (0) |
| G7 | Virginiamycin 2ppm | 0/15 (0) | 0/15 (0) | 0/30 (0) |

### 3.2 Mean Body Weight and Weight Gain

Following the *Clostridium perfringens* challenge (days 14-16), the mean body weights of the OxBC treatment groups were significantly *(P* <*0.05)* increased relative to the challenge-non-medicated bird group (G2). The 2 ppm OxBC group (G3) had the highest overall weight gain (days 1 to 28) among the three OxBC doses. Treatment with OxBC restored the final average body weight and overall average weight gain of challenged birds to levels that were not significantly different than that observed for the non-challenged controls (G1) (Table 8).

**Table 8. Mean body weight and percent weight gain**

| **Groups (n=30)** | | **Mean body weight (g)^{†}** | | | | **%Weight gain**^{††} | | |
|---|---|---|---|---|---|---|---|---|
| | | **1-d** | **14-d** | **21-d** | **28-d** | **1 to 14d** | **14 to 28d** | **1 to 28d** |
| G1 | Non-challenged control | 45. 0 | 460.2^{b}±31. 2 | 870.2^{b*}±58. 9 | 1445.0^{b*}±73.8 | 922.6 | 214.0 | 3111.0 |
| G2 | Non-medicated control | 44. 8 | 453.4±31. 1 | 810.6^{a}±58.3 | 1275.0^{a}±84.7 | 911.9 | 181.2 | 2745.9 |
| G3 | OxBC 2ppm | 44. 8 | 459.9^{b}±27. 8 | 855.0^{b*}±57. 3 | 1422.6^{b*}±85.9 | 926.5 | 209.4 | 3075.4 |
| G4 | OxBC 4ppm | 44. 7 | 460.8^{b}±37. 4 | 845.1^{b*}±51. 4 | 1415.8^{b*}±83.3 | 930.8 | 207.3 | 3067.2 |
| G5 | OxBC 6ppm | 44. 5 | 441.0^{a}±22. 0 | 846.9^{b*}±39. 6 | 1412.5^{b*}±98.5 | 890.9 | 220.3 | 3074.0 |
| G6 | Bacitracin 55p pm | 44. 4 | 462.3^{b}±26. 0 | 960.0^{c**}±70 .1 | 1627.7^{c**}±117 .6 | 941.1 | 252.1 | 3566.0 |
| G7 | Virginiamycin 2ppm | 44. 5 | 500.5^{c*}±32. 4 | 950.5^{c**}±80 .0 | 1616.6^{c**}±105 .9 | 1024.7 | 223.0 | 3532.8 |

| | | | | | | | | |
|---|---|---|---|---|---|---|---|---|
| ^{†} The superscripts ^{a, b, c} denotes a significant difference statistically. *, *P* < 0.05; ***, P* < 0.001, One-Way ANOVA (SPSS 12.0). ^{††} Percent weight gain = [(final weight - initial weight) / initial weight] × 100. | | | | | | | | |

### 3.3 Intestinal Lesion Scores

Macroscopic (gross) lesions in the small intestine were evaluated at 3 time points during the study:
- On study day-10, prior to challenge (on days 14-16), five birds from each isolator were euthanized and evaluated for pre-challenge hyperemia and hemorrhages.
- On study day-21, five days after the final CP challenge, three birds from each isolator were euthanized and evaluated for intestinal lesions.
- On study day-28, the final day of the study, the remaining 12 birds in each incubator were evaluated for intestinal lesions.

Evaluation of pre-challenge samples taken on day-10 revealed that there was little to no multifocal hyperemia and/or hemorrhages present on the intestinal mucosa in any of the treatment groups. This finding confirmed that birds in all groups were in good health prior to the challenge period that began on day-14.

The *C. perfringens* challenge induced lesions in the small intestine. The challenged-non-medicated group (G2) had the highest mean lesion score (1.54±0.58), which was significantly higher than scores for any other group in the study (Table 9). The non-challenge group (G1) had the lowest mean lesion score of 0.30±0.39.

During the post-challenge period (days 14 to 28) treatment with OxBC significantly alleviated intestinal lesions compared to the challenged-non-medicated group. Remarkably, mean lesion score of birds fed 2 ppm OxBC was the lowest (0.40±0.51) of all groups except the non-challenged control group (Table 9).

Mean lesion scores were also significantly reduced in both antibiotic groups relative to challenged-non-medicated controls (Table 9).

**Table 9. Intestinal lesion scores (3 birds on day 21 & 12 birds on day 28)**

| **Groups** | | **Mean lesion scores^{†}** | | |
|---|---|---|---|---|
| | | **Replicate 1 (n=15)** | **Replicate 2 (n=15)** | **Mean (n=30)** |
| G1 | Non-challenged control | 0.07^{a*}±0.26 | 0.53^{a*}±0.52 | 0.30^{a*}±0.39 |
| G2 | Non-medicated control | 1.47^{d}±0.52 | 1.60^{b}±0.63 | 1.54^{b}±0.58 |
| G3 | OxBC 2ppm | 0.47^{abc*}±0.52 | 0.33^{a*}±0.49 | 0.40^{a*}±0.51 |
| G4 | OxBC 4ppm | 0.73^{c*}±0.80 | 0.33^{a*}±0.49 | 0.53^{a*}±0.65 |
| G5 | OxBC 6ppm | 0.67^{bc*}±0.62 | 0.33^{a*}±0.49 | 0.50^{a*}±0.56 |
| G6 | Bacitracin 55ppm | 0.27^{ab*}±0.46 | 0.67^{a*}±0.62 | 0.47^{a*}±0.54 |
| G7 | Virginiamycin 2ppm | 0.40^{abc*}±0.51 | 0.60^{a*}±0.63 | 0.50^{a*}±0.57 |

| | | | | |
|---|---|---|---|---|
| ^{†} The superscripts a, b, c denotes a significant difference statistically. **, P* < 0.05; ***, P* < 0.001, One-Way ANOVA (SPSS 12.0). Standard deviation was calculated with mean value of each group | | | | |

### 3.4 Enumeration of Clostridium perfringens

The *C. perfringens* content of fecal samples collected from the small intestine was evaluated, by determination of colony forming units (CFU), at three time points during the study.
- On study day-10, prior to challenge (on days 14-16), five birds from each isolator were euthanized and evaluated for fecal *C. perfringens* content.
- On study day-21, five days after the final *C. perfringens* (*"CP")* challenge, three birds from each isolator were euthanized and evaluated for fecal *C. perfringens* content.
- On study day-28, the final day of the study, the remaining 12 birds in each incubator were evaluated for fecal *C. perfringens* content.

The pre-challenge level of *C. perfringens,* determined on day-10, ranged from 4.4 x 10² to 1.3 x 10³ CFU across the seven treatment groups (Table10). This low level of *C. perfringens* is expected as this species is commonly found to reside in the gut of broilers at low, non-pathogenic levels.

For the non-challenged control group *C. perfringens* levels increased throughout the trial. As indicated above *C. perfringens* is a normal resident of the broiler gut microflora and at low levels the bacteria do not affect the bird. It is also not uncommon for the intestinal content of *C. perfringens* to increase as the birds age. The *C. perfringens* levels observed in the non-challenge control throughout this study were consistent with the non-pathological levels normally observed in healthy birds.

In the post-challenge period, on days 21 and 28, birds in the challenge + non-medicated group had *C. perfringens* levels that were significantly higher than all other groups (Table 10). Treatment with OxBC significantly and dose-dependently reduced *C. perfringens* levels by 2 to 3 orders of magnitude relative to the challenged non-medicated group at each post-challenge time point evaluated (days 21 and 28). Furthermore, *C. perfringens* levels in the OxBC groups were not statistically different than those of the non-challenged birds (Table 10).

Use of antibiotics also resulted in significant reductions in *C. perfringens* levels during the post-challenge period. By the end of the study, on day 28, no *C. perfringens* was recovered from samples taken from birds in either antibiotic group. The complete abolishment of *C. perfringens* by the antibiotics is in contrast to the effects of OxBC, which maintained *C. perfringens* levels at the normal non-challenge levels (Table 10).

**Table 10. Enumeration of Clostridium perfringens in the small intestine of broiler chickens**

| **Groups** | | **Mean CFU per gram^{†} of chicken feces on** | | |
|---|---|---|---|---|
| | | **Day 10 (*n*=10)** | **Day 21 (*n*=6)** | **Day 28 (*n*=24)** |
| G1 | Non-challenged control | 4.4×10²±8.9×10 | ¹ 1.0x10^{3[a*]}±2.7×10² | 8.3×10^{5[a**]}±2.0×10⁵ |
| G2 | Non-medicated control | 1.0×10³±2.7×10² | 2.0×10^{5[b]}±5.0×10⁴ | 1.1x10^{7[b]}±9.2×10⁵ |
| G3 | OxBC 2ppm | 1.0×10³±2.8×10² | 7.5×10^{3[a*]}±1.9×10³ | 1.7×10^{5[a*]}±2.4×10 ⁴ |
| G4 | OxBC 4ppm | 4.5×10²±1.3×10² | 1.2×10^{3[a*]}±2.0×10² | 3.4×10^{4[a**]}±7.3×10 ³ |
| G5 | OxBC 6ppm | 1.3×10³±2.3×10² | 2.3×10^{2[a*]}±5.7×10¹ | 7.1×10^{4[a**]}±1.4×10 ⁴ |
| G6 | Bacitracin 55ppm | 8.6×10²±1.6×10² | 2.5×10¹±9.6 | 0.0 |
| G7 | Virginiamycin 2ppm | 1.1×10³ ± 2.2×10² | 0.0 | 0.0 |

| | | | | |
|---|---|---|---|---|
| ^{†}The superscripts ^{[a,b]} denotes a significant difference statistically.*, *P* < 0.05; **, *P* < 0.001, One-Way ANOVA (SPSS 12.0). Mean values of antibiotic treatment groups (Groups 6 and 7) were excluded from the statistical analysis because the number of Clostridial bacteria was not very low or detected. Standard deviation was calculated with mean value of each group. | | | | |

### 4. Study Summary

- This study assessed the preventive effect of OxBC in a subclinical model of necrotic enteritis in the broiler chicken. Several parameters were evaluated, including survival (mortality) rate, clinical signs, body weight, weight gain, intestinal lesion, and bacterial enumeration. OxBC was evaluated relative to a challenged-non-medicated control group, a non-challenged control group, two antibiotic groups (Bacitracin & Virginiamycin).
- No mortality was observed during the trial for any of the groups. This is because the study employed a subclinical necrotic enteritis model that was intended to present clinical signs or pathological indicators rather than high mortality rate characteristic of acute or clinical models of the disease.
- In the post-challenge period, on days 21 and 28, birds in all OxBC groups had significantly higher mean body weights *(P* < *0.05)* compared to the challenged-non-medicated control group.
- The severity of gross lesions observed during the trial were consistent with the subclinical level of the challenge model. Evaluation of lesions on days 21 and 28 revealed the presence of various pathological findings, such as disseminated severe hyperemia and hemorrhages, focal necrosis or ulceration of the intestinal mucosa of birds in the challenged-non-medicated control group. In contrast, birds in the OxBC and antibiotic groups showed lesion scores that were reduced by approximately 3-fold compared to the challenged-non-medicated group. Furthermore, the severity of the lesions in the OxBC and antibiotic groups did not differ significantly from those observed in the non-challenge control birds. The improvement in intestinal lesion scores occurred concurrent with a reduction in *C. perfringens* levels in the feces of birds fed OxBC or antibiotics.

### 5. Conclusion

This study demonstrates the ability of low part-per-million levels of OxBC in the feed to protect against the deleterious effects of necrotic enteritis on broiler health and productivity. The benefits of OxBC were observed at the level of growth performance (body weight and weight gain), intestinal health (reduced severity of NE lesions), and pathogen colonization (reduced *C. perfringens* levels in the small intestine). The fact that birds receiving OxBC performed as well as healthy non-challenged controls is further evidence of the product's protective effects.

In one embodiment, The use of 2 to 6 ppm dietary OxBC supplementation as a feed additive in commercial chicken farms can contribute to the prevention and improvement of chicken NE and is expected to have a positive effect in improving productivity during breeding period.

### EXAMPLE 4 -Evidence that Improved Measures of Productivity in Food Animals by Fully Oxidized β-Carotene are Associated with Immunological Activity and that this is a Characteristic Feature of Fully Oxidized Carotenoids.

### The Starting Point - Carotenoids

Carotenoids are yellow, orange, and red pigments synthesized by plants. There are over 600 known carotenoids that are made up of two classes called carotenes, which are purely hydrocarbons, and xanthophylls, which are carotenes substituted with one or a few oxygen atoms. β-Carotene, and lycopene are examples of common carotenes, whereas lutein, zeaxanthin, and canthaxanthin are common examples of xanthophylls. The most common carotenoids in North American diets are α-carotene, β-carotene, β-cryptoxanthin, lutein, zeaxanthin, and lycopene.

All carotenoids are formed from 8 isoprene units and each carotenoid molecule contains 40 carbon atoms. Structurally, carotenoids take the form of a polyene hydrocarbon chain, which is sometimes terminated at one or both ends by a ring. Carotenoids that contain unsubstituted β-ionone rings (including β-carotene, α-carotene, β-cryptoxanthin and γ-carotene) have vitamin A activity (meaning that they can be converted to retinal). By contrast, lutein, zeaxanthin, and lycopene have no vitamin A activity.

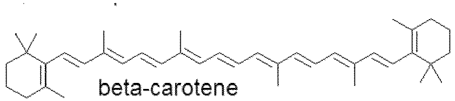

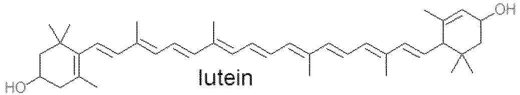

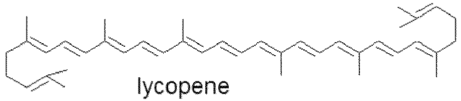

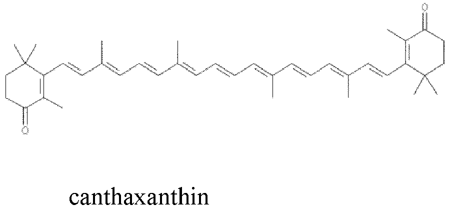

The colour of carotenoids, ranging from pale yellow through bright orange to deep red, is directly linked to their structure. The carbon-carbon double bonds interact with each other in a process called conjugation, which allows electrons in the molecule to move freely across these areas of the molecule and to readily undergo electronic transitions by absorbing energy in the visible light region.

### The End Point - Oxygen Spontaneously Forms Co-Polymers with Carotenoids as the Main Reaction Product

The very same system of conjugated double bonds that gives rise to the intense colour of carotenoids also makes them highly susceptibility to spontaneous reaction with molecular oxygen. The present inventors discovered that the oxidation reaction occurs predominantly by *addition* of multiple oxygen molecules to the carotenoid molecule to form carotenoid-oxygen copolymer products [1, 2 and 11]. For example, β-carotene dissolved in a suitable organic solvent (e.g., benzene, ethyl acetate), reacts with almost 8 equivalents of molecular oxygen resulting in a net weight increase of ca. 30%. The product, OxBC, is made up mainly of polymers (85% by weight) with norisoprenoid breakdown products (15%) providing the balance.

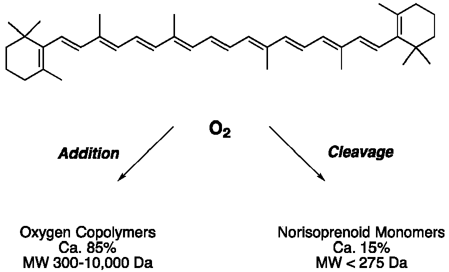

The spontaneous reaction of β-carotene with oxygen proceeds predominantly by addition to form polymers with a high content of oxygen. OxBC is the total reaction product and is comprised of both polymer (85%) and cleavage products (15%). Other carotenoids react similarly, with a preponderance of polymer product formation.

The dominance of the polymeric product also is reflected in the empirical formula of OxBC (Table), which differs only slightly from the empirical formula of its isolated polymer product and is consistent with the addition of multiple molecules of O₂ per β-carotene molecule. The gel phase permeation chromatogram (GPC) (i.e., size-exclusion chromatogram) of OxBC is dominated by a broad peak extending up to *ca.* 8,000 Da. with a median MW of 900 Da. **(****Fig. 2A****)**

The extended system of linear conjugated double bonds present in β-carotene is common to all carotenoids so it is expected that other carotenoids will behave similarly in their spontaneous reactions with molecular oxygen. The Table below shows that this expectation is borne out, as illustrated by comparing the results of the full oxidations of β-carotene, lycopene, canthaxanthin and lutein.

Comparison of the results of the spontaneous, full oxidation by oxygen of β-carotene, lycopene, canthaxanthin and lutein are illustrated in the following table:

**Table 11**

| | β-Carotene | Lycopene | Canthaxanthin | Lutein |
|---|---|---|---|---|
| Starting carotenoid molecular formula | C₄₀H₅₆ | C₄₀H₅₆ | C₄₀H₅₂O₂ | C₄₀H₅₆O₂ |
| Fully oxidized carotenoid | OxBC | OxLyc | OxCan | OxLut |
| Empirical formula - total product | C₄₀H₆₀O₁₅ | C₄₀H₆₁O₂₀ | C₄₀H₅₆O₁₃ | C₄₀H₅₉O₁₄ |
| Empirical formula - isolated polymer | C₄₀H₅₉O₁₈ | C₄₀H₅₈O₂₀ | C₄₀H₅₁O₁₆ | C₄₀H₅₆O₁₅ |
| Polymer - proportion of product | 85% | >80% | >80% | >80% |
| O₂ consumed (equivalents) | 6-8 | n.d. | 7 | n.d. |
| Weight increase | 30% | ∼37% | 25% | 25% |
| GPC analysis (Fig. 3) | ✔ | ✔ | ✔ | ✔ |
| Infra red spectra (FTIR; Fig. 4) | ✔ | ✔ | ✔ | ✔ |

| | | | | |
|---|---|---|---|---|
| n.d. - not determined. | | | | |

In common with the oxidation of β-carotene, these carotenoids all show high uptake of oxygen, as reflected in the overall substantial increase (≥25%) in product weight and the similar empirical formulae, with a predominance of polymer products, as shown by their GPCs **(****Figs. 2B-3D****).** Furthermore, all of the fully oxidized carotenoids have strikingly similar infrared spectra (FTIR) (Figs. 3A-3D).

### Enhancing Immune Function - the Example of Fully Oxidized β-Carotene (OxBC)

The inventors have found that OxBC is capable of exerting an unusual dual capability upon immunological function [3-5]. The two effects are (1) an ability to prime and enhance innate immune function [2,3] and (2) to limit excess inflammation [12]. These effects are independent of any vitamin A activity because OxBC is free of both β-carotene and vitamin A [3], and, furthermore, has been shown to lack the ability to activate retinoic acid receptors [3].

The discovery of this activity stems from recognizing the polymeric products β-carotene predominantly forms spontaneously in reaction with molecular oxygen [2] and that these products are primarily responsible for the observed immunological activities [3] (see below).

As this type of oxidation behaviour is not confined to β-carotene alone but is characteristic of carotenoids in general (see above), these observations provide a tangible, credible and testable basis for explaining the non-vitamin A effects of both the provitamin A carotenoids (α-, β- and γ-carotenes and β-cryptoxanthin) and the more numerous carotenoids that cannot be converted into vitamin A.

Preliminary screening of OxBC using a PCR gene expression array showed a pattern of activity indicating the potential for the two key immunomodulatory capabilities (Table 3 in ref. [2]). In the first capability, relating to modulation of innate immune function, OxBC up-regulates the expression of genes encoding products that function in pathogen sensing and the detection of pathogen-associated molecule patterns (PAMPs), including toll-like receptors (TLRs) and other proteins that act as cofactors for PAMP detection, such as CD-14 (cluster of differentiation 14).

An ability to prime innate immune function has been corroborated both *in vitro* and *in vivo,* as indicated by increased expression of plasma membrane TLR and CD14 receptors (Figs.1 and 2 in ref. [3]). Furthermore, as will be described below, an assay based on CD14 receptor expression indicates the polymer product is principally, if not wholly, responsible for OxC-beta's activity.

### Extension to Other Fully Oxidized Carotenoids - Demonstrating Increased Levels of Immune Surveillance Receptors by Fully Oxidized Lycopene

Central to OxBC's immune enhancing actions is its ability to increase the level of pathogen-sensing immune receptors, including toll-like receptors subtypes 2 and 4 (TLR-2 and TLR-4) and CD14. These receptors play the vital role of pathogen detection and activation of the innate immune system. By increasing the host's complement of immune receptors OxBC effectively heightens the level of immune surveillance for pathogens.

To assess the ability of other carotenoids to enhance innate immunity, an assay was developed based on upregulation of CD14 immune surveillance receptors [3]. Fully oxidized lycopene (OxLyc) was compared with fully oxidized β-carotene (OxBC). Although lycopene shares the same number of linearly conjugated double bonds as β-carotene, it lacks the cyclohexyl groups at each end.

**Fig. 4A** shows a linear dose-response of CD14 expression to concentration of OxBC and OxLyc, respectively, and that their activities are essentially indistinguishable. The comparison of the lycopene product with the β-carotene product indicates a lack of influence of the cyclohexyl groups upon CD14 immune receptor response.

### Carotenoid-Oxygen Copolymers are Responsible for Enhancing Innate Immunity

**Fig 4B** shows that it is the polymeric fraction of OxBC that is principally responsible for upregulating CD14 expression. This finding and the essentially equivalent activities of OxBC and OxLyc in the CD14 assay are consistent with the predominance of the polymeric product in each of the oxidized carotenoids and the strong similarity between their FTIR spectra, indicating the existence of common structural elements in the oxidized carotenoid polymers.

CD14 expression was quantified using FACS analysis. The effect of each compound is shown relative to untreated cells. Points represent the mean and standard error from three separate experiments. (A) OxLyc had a significant dose effect on CD14 surface content (p = 0.020) that was not significantly different from the effect of OxBC. (B) Correlation analysis indicates a significant dose effect for each compound on CD14 expression with p-values of 0.0036 for OxBC, 0.0034 for the polymer, and 0.0113 for the monomer. Comparison of the relative activity of each compound (B) indicates that the monomer is significantly less active than the polymer (p < 0.001) and OxBC (p < 0.01) while there is no significant difference between the activities of the polymer and OxBC. The apparent activity of the monomer may be due to the presence of residual lower molecular weight polymers that could not be completely removed from the monomer fraction (from ref. [3]).

### The Importance of Enhancing Innate Immunity to Livestock Production

Modern livestock lines have been selected for optimal growth, feed conversion efficiencies and production levels. Concomitant with the genetic selection for higher production potentials has been the reduction in the animals' metabolically costly, immunological potential. As a consequence modern livestock species are susceptible to infection. This susceptibility coupled with intense production environments renders food animals vulnerable to infection from numerous pathogens. Reduction in growth rates, feed conversion efficiencies, and increased mortalities brought on by infections pose a significant economic threat to the livestock industry worldwide. To try and mitigate the threat that infections pose the industry has turned to the development of feed ingredients with an ability to enhance the host's *innate immune* defenses. OxBC represents one such feed ingredient with the ability to beneficially impact the host's innate immune system.

In this scenario, the importance of OxBC's immune enhancing actions can be seen. Through its ability to increase the level of pathogen sensing immune receptors, including TLR-2. TLR-4 and CD14, OxBC can reinforce the vital role of pathogen detection and activation of the innate immune system. By increasing the host's complement of immune receptors OxBC effectively heightens the level of immune surveillance for pathogens.

This heightened surveillance leads to an earlier stage detection, reaction, and clearance of pathogens. Immunological responses are metabolically costly and can take resources away from growth and production pathways. Through its effects on immune receptor expression OxBC provides a mechanism for preventing infections at an early stage thereby limiting the scope and duration of the immunological response and sparing metabolic resources. As a result the animal is maintained in a healthy state and metabolic resources can be conserved and directed towards attaining the animal's full growth potential.

While the foregoing invention has been described in some detail for purposes of clarity and understanding, it will be appreciated by one skilled in the art, from a reading of the disclosure, that various changes in form and detail can be made without departing from the true scope of the invention in the appended claims.

### REFERENCES

1. Burton, GW, Daroszewski, J, Phipps, J. 1995. Extensively oxidized derivatives of carotenoids, retinoids and related conjugated polyenes useful as non-toxic cell-differentiation inducers, anti-proliferative agents, and anti-tumor agents. US Patent 5,475,006.
2. Burton, GW, Daroszewski, J, Nickerson, JG, Johnston, JB, Mogg, TJ, Nikiforov, GB. 2014. β-Carotene autoxidation: oxygen copolymerization, non-vitamin A products and immunological activity. Can. J. Chem. 92: 305-316.
3. Johnston JB, Nickerson JG, Daroszewski J, Mogg TJ, Burton GW. 2014. Biologically active polymers from spontaneous carotenoid oxidation: a new frontier in carotenoid activity. PLoS ONE 9 e111346.
4. Cui B, Liu S, Wang Q, Lin X. 2012. Effect of β-carotene on immunity function and tumour growth in hepatocellular carcinoma rats. Molecules 17: 8595-8603.
5. Shojadoost B, Vince AR, Prescott JF. 2012. The successful experimental induction of necrotic enteritis in chickens by Clostridium perfringens: a critical review. Veterinary research 43: 74.
6. Prescott JF, Sivendra R, Barnum DA. 1978. The use of bacitracin in the prevention and treatment of experimentally-induced necrotic enteritis in the chicken. The Canadian veterinary journal 19: 181-183.
7. Gholamiandehkordi AR, Timbermont L, Lanckriet A, Van Den Broeck W, Pedersen K, Dewulf J, Pasmans F, Haesebrouck F, Ducatelle R, Van Immerseel F. 2007. Quantification of gut lesions in a subclinical necrotic enteritis model. Avian pathology 36, 375-382.
8. Chen CY, Nace GW, Irwin PL. 2003. A 6 x 6 drop plate method for simultaneous colony counting and MPN enumeration of Campylobacter jejuni, Listeria monocytogenes, and Escherichia coli. Journal of microbiological methods 55, 475-479.
9. Yan F, Zhao Y, Hu Y, Qiu J, Lei W, Ji W, Li X, Wu Q, Shi X, Li Z. 2013. Protection of chickens against infectious bronchitis virus with a multivalent DNA vaccine and boosting with an inactivated vaccine. J Vet Sci. 14:53-60.
10. Antão EM, Glodde S, Li G, Sharifi R, Homeier T, Laturnus C, Diehl I, Bethe A, Philipp HC, Preisinger R, Wieler LH, Ewers C. 2008. The chicken as a natural model for extraintestinal infections caused by avian pathogenic Escherichia coli (APEC). Microb Pathog. 45:361-369.
11. Burton, G.W., et al., Oxidized carotenoids, retinoids and related conjugated polyenes, and derived fractions and compounds useful as cell-differentiation inducers, cytostatic agents, and anti-tumor agents, in WO 96/05160, W.I.P. Organization, 1996.
12. Duquette, S.C., et al., Anti-inflammatory benefits of retinoids and carotenoid derivatives: retinoic acid and fully oxidized β-carotene induce caspase-3-dependent apoptosis and promote efferocytosis of bovine neutrophils. Am J Vet Res, 2014. 75.

## Claims

1. Fully oxidized beta-carotene for use in reducing the severity of intestinal lesions associated with Necrotic Enteritis (NE) in poultry, wherein the use comprises administering an effective amount of the fully oxidized beta-carotene to said poultry.

2. The fully oxidized beta-carotene for use according to claim 1, wherein the poultry is a broiler chicken.

3. The fully oxidized beta-carotene for use according to claim 1, wherein the intestinal lesions are caused by C. perfringens.

4. The fully oxidized beta-carotene for use according to any one of claims 1 to 3, wherein the effective amount is 2 - 30 ppm of diet.

5. A composition comprising fully oxidized beta-carotene for use in reducing the severity of intestinal lesions associated with Necrotic Enteritis in poultry, wherein the composition is an animal feed.

6. The composition for use according to claim 5, wherein the animal feed is poultry feed.

## Patentansprüche

1. Vollständig oxidiertes beta-Carotin zur Verwendung bei der Reduzierung des Schweregrades von mit nekrotischer Enteritis (NE) assoziierten intestinalen Läsionen in Geflügel, wobei die Verwendung die Verabreichung einer wirksamen Menge des vollständig oxidierten beta-Carotins an das Geflügel umfasst.

2. Vollständig oxidiertes beta-Carotin zur Verwendung nach Anspruch 1, wobei es sich bei dem Geflügel um ein Masthähnchen handelt.

3. Vollständig oxidiertes beta-Carotin zur Verwendung nach Anspruch 1, wobei die intestinalen Läsionen durch C. perfringens verursacht werden.

4. Vollständig oxidiertes beta-Carotin zur Verwendung nach einem der Ansprüche 1 bis 3, wobei die wirksame Menge 2-30 ppm der Nahrung beträgt.

5. Zusammensetzung, umfassend vollständig oxidiertes beta-Carotin zur Verwendung bei der Reduzierung des Schweregrades von mit nekrotischer Enteritis assoziierten intestinalen Läsionen in Geflügel, wobei es sich bei der Zusammensetzung um ein Tierfutter handelt.

6. Zusammensetzung zur Verwendung nach Anspruch 5, wobei es sich bei dem Tierfutter um Geflügelfutter handelt.

## Revendications

1. Bêta-carotène totalement oxydé destiné à être utilisé dans une réduction de la gravité de lésions intestinales associées à une entérite nécrotique (EN) chez une volaille, dans lequel l'utilisation comprend l'administration d'une quantité efficace du bêta-carotène totalement oxydé à ladite volaille.

2. Bêta-carotène totalement oxydé destiné à être utilisé selon la revendication 1, dans lequel la volaille est un poulet d'élevage.

3. Bêta-carotène totalement oxydé destiné à être utilisé selon la revendication 1, dans lequel les lésions intestinales sont causées par C. perfringens.

4. Bêta-carotène totalement oxydé destiné à être utilisé selon l'une quelconque des revendications 1 à 3, dans lequel la quantité efficace est de 2 - 30 ppm de régime alimentaire.

5. Composition comprenant du bêta-carotène totalement oxydé destiné à être utilisé dans une réduction de la gravité de lésions intestinales associées à une entérite nécrotique chez une volaille, la composition étant un aliment pour animaux.

6. Composition destinée à être utilisée selon la revendication 5, dans laquelle l'aliment pour animaux est un aliment pour volailles.
